# EUROPÄISCHE PATENTANMELDUNG

(11) **EP 0 551 916 A2**
(43) Veröffentlichungstag der Anmeldung: **21.07.1993**
(21) Anmeldenummer: 93100579.7
(22) Anmeldetag: 15.01.1993
(51) Int. Cl.: C07K 3/08, C12N 9/96, C07K 3/00

(54) **Verfahren zur Stabilisierung von Proteinen**

(30) Priorität: 17.01.1992 DE 4201181
(71) Anmelder: BOEHRINGER MANNHEIM GMBH, D-68298 Mannheim (DE)
(72) Erfinder: Jakob, Ursula, W-8400 Regensburg (DE); Buchner, Johannes, Dr., W-8400 Regensburg (DE); Wiech, Hans, W-2855 Beverstedt (DE); Zimmermann, Richard, Prof. Dr., W-3400 Göttingen (DE); Rudolph, Rainer, Prof. Dr., W-8120 Weilheim (DE)
(74) Vertreter: Huber, Bernhard, Dipl.-Chem.

(57) **Zusammenfassung**

Die vorliegende Erfindung betrifft ein Verfahren zur Stabilisierung von Proteinen in einer wäßrigen Lösung, welches dadurch gekennzeichnet ist, daß man der wäßrigen proteinhaltigen Lösung ein oder mehrere Vertreter aus der "Hsp90" Proteinfamilie zusetzt.

## Beschreibung

Die vorliegende Erfindung betrifft ein Verfahren zur Stabilisierung von Proteinen in einer wäßrigen Lösung durch Zusatz einer als Stabilisator wirkenden Proteinkomponente.

Proteine der Hsp (Heat Shock Protein) 90 Familie, die im aktiven Zustand im allgemeinen als Dimere vorliegen, sind sowohl in prokaryontischen als auch in eukaryontischen Organismen bekannt. Das aus E.coli isolierte Hsp90-homologe Protein wird als Htp G bezeichnet (Bardwell & Graig, Proc.Natl. Acad.Sci. USA 84 (1987), 5177-4181). Weiterhin ist beispielsweise in Drosophila melanogaster ein Hsp90 Genprodukt bekannt (Blackman und Meselson, J.Mol.Biol. 188 (1986), 499-515), während beim Menschen mehrere Hsp90 Genprodukte bekannt sind (Simon et al., Mol.Cell. Biol. 7 (1987), 2884-2890; Sorger et al., Nature 329 (1987), 81-84).

Ein Sequenzvergleich von Proteinen der Hsp90 Familie zwischen verschiedenen Organismen ergibt starke Homologien. Die Proteine der evolutionär am weitest entfernten eukaryontischen Organismen zeigen auf Aminosäureebene noch etwa 50 % Übereinstimmung. Die Homologien zwischen den entsprechenden Proteinen aus Eukaryonten und Prokaryonten liegen bei etwa 40 % (Bardwell und Craig, Proc.Natl.Acad.Sci. USA 84 (1987), 5177-4181).

Alle bisher bekannten eukaryontischen Vertreter der Hsp90 Familie weisen an ähnlicher Position im N-Terminus eine stark negativ geladene Region auf, die bezüglich der Aminosäuresequenz nur wenig konserviert ist. Sowohl in eukaryontischen als auch in prokaryontischen Hsp90 Molekülen befindet sich am C-Terminus ein weiterer stark negativ geladener Bereich, der bis auf die letzten vier Aminosäuren ebenfalls nicht konserviert ist. Diese Sequenz Glu-Glu-Val-Asp wurde bisher in allen bekannten Vertretern der Hsp90 Familie gefunden.

Zur Funktion von Hsp90 ist bisher bekannt, daß es in vivo mit verschiedenen Tyrosinkinasen, z.B. retroviralen Kinasen (Brugge et al., Cell 25 (1981), 363-372; Lindquist und Craig, Annu.Rev. Gene 22 (1988), 631-677) sowie mit der Häm-regulierten zellulären elF2α-Kinase (Rose et al., Biochemistry 26 (1987), 6583-6587) in Wechselwirkung tritt und deren Phosphorylierungsgrad und Aktivität beeinflussen kann.

Ferner ist bekannt, daß Vertreter der Hsp90-Familie an Steroidhormon-Rezeptor-Komplexe binden können (Pratt et al., J.Biol.Chem. 263 (1988), 267-273; Welch (1990), in: "Stress Proteins in Biology and Medicine", Herausgeber: Morimoto, R.I., Tissiéres, A. & Georgopoulos, C., Cold Spring Harbor Laboratory Press, Cold Spring Harbor, New York, S. 223-278).

Hsp90 kann auch an Aktin binden und Quervernetzungen zwischen Aktinfilamenten ausbilden (Koyasu et al., Proc.Natl.Acad.Sci. USA 83 (1986), 8054-8058). Schließlich ist noch bekannt, daß Hsp90 die Dissoziation eines hormonfreien Glucocorticoidrezeptors aus einem DNA-Rezeptor-Komplex bewirken kann (Scherrer et al., J.Biol.Chem. 265 (1990), 21397-21400).

Eine Stabilisierung von Proteinen durch Zusatz von unterschiedlichen als Stabilisator wirkenden Proteinkomponenten ist bekannt. Die Nachteile bekannter Stabilisatorproteine bestehen jedoch darin, daß sie nur in Gegenwart von ATP wirksam sind, in großem molekularem Überschuß eingesetzt werden müssen oder/und selbst komplizierte, aus über 10 Untereinheiten zusammengesetzte Oligomere darstellen.

Die Aufgabe der vorliegenden Erfindung bestand somit darin, ein neues Verfahren zur Stabilisierung von Proteinen in einer wäßrigen Lösung bereitzustellen, bei dem diese oben genannten Nachteile mindestens teilweise beseitigt sind.

Die erfindungsgemäße Aufgabe wird durch die Bereitstellung eines Verfahrens zur Stabilisierung von Proteinen in einer wäßrigen Lösung gelöst, welches dadurch gekennzeichnet ist, daß man der wäßrigen proteinhaltigen Lösung einen oder mehrere Vertreter aus der "Hsp90" Proteinfamilie zusetzt.

Es wurde überraschenderweise festgestellt, daß "Hsp90" Proteine andere Proteine in einer wäßrigen Lösung stabilisieren können, wobei diese stabilisierende Wirkung insbesondere auf eine Verhinderung der Bildung von unlöslichen Aggregaten beruht. Das erfindungsgemäße Verfahren kann beispielsweise zur Unterstützung bei der in vitro Faltung von denaturierten Proteinen, aber auch zur Stabilisierung von nativen Proteinen in gelöstem Zustand verwendet werden.

Das erfindungsgemäße Verfahren kann bei beliebigen Proteinen angewandt werden. Vorzugsweise wird es bei solchen Proteinen verwendet, die wegen ihrer Temperaturempfindlichkeit oder aus sonstigen Gründen in gelöstem Zustand leicht zur Aggregation neigen. Ein wichtiges Anwendungsgebiet für das erfindungsgemäße Verfahren ist die Stabilisierung von Lösungen, die enzymatisch aktive Proteine (z.B. die Citratsynthase) enthalten.

Ein weiteres wichtiges Anwendungsgebiet ist die Stabilisierung von Lösungen, die Antikörper oder von Antikörpern abgeleitete Derivate (z.B. Fab- oder F(ab)₂-Fragmente) enthalten.

Die "Hsp90" Proteine bewirken eine Stabilisierung von Proteinen bei an sich beliebigen Proteinkonzentrationen, wobei jedoch eine besonderes gute Stabilisierungswirkung in einem Konzentrationsbereich von 0,0001 µmol/l bis 100 µmol/l des zu stabilisierenden Proteins erzielt wird.

Das molare Verhältnis zwischen dem zu stabilisierenden Protein und dem zugesetzten "Hsp90" Protein beträgt bei dem erfindungsgemäßen Verfahren vorzugsweise 0,0001 : 1 bis 10 : 1. Dieses molare Verhältnis bezieht sich dabei auf den im allgemeinen zwei Untereinheiten aufweisenden Hsp90-Komplex. Besonders bevorzugt wird das "Hsp90" Protein in einem molaren Verhältnis von 0,001 : 1 bis 5 : 1 bezüglich der zu stabilisierenden Proteine zugesetzt.

Ein besonderer Vorteil des erfindungsgemäßen Verfahrens besteht darin, daß die Stabilisierung der Proteine in Abwesenheit von ATP erfolgen kann, bzw. daß auch ein Zusatz von ATP im allgemeinen keinen Einfluß auf die Stabilisierungswirkung des "Hsp90" Proteins zeigt. Auf diese Weise ist es ohne weiteres möglich, Proteinlösungen für enzymatische Tests zu stabilisieren, bei denen ATP als Cosubstrat eingesetzt wird, ohne daß dadurch ein störender Einfluß auf den enzymatischen Test auftritt.

Die erfindungsgemäße Stabilisierung von Proteinen wird mit "Hsp90" Proteinen beobachtet, die aus prokaryontischen oder eukaryontischen Organismen stammen können. Die Reinigung des "Hsp90" Proteins aus Rinderpankreas erfolgt beispielsweise nach dem von Welch und Feramisco (J.Biol.Chem. 257, (1982) 14949-14959) beschriebenen Verfahren. Eine Auflistung weiterer "Hsp90" Proteine, die zu diesem Protein homolog sind und die für das erfindungsgemäße Verfahren eingesetzt werden können, findet man in Lindquist, S.C. und Craig, E.A. (1988), Ann. Rev. Genet. 22: 631-677.

Eine bevorzugte Anwendung des erfindungsgemäßen Verfahrens besteht in der Unterstützung bei einer in vitro Faltung bzw. Renaturierung von denaturierten Proteinen. Dabei handelt es sich vorzugsweise um Proteine, die durch heterologe Expression in Prokaryonten synthetisiert worden sind und dabei im allgemeinen in Form von Inclusion Bodies anfallen. Vorzugsweise handelt es sich bei den heterolog exprimierten Proteinen um eukaryontische Proteine. Der Begriff "heterologe Expression" bedeutet in diesem Zusammenhang, daß ein Protein aus einem fremden (heterologen) Organismus oder/und unter Kontrolle eines fremden (heterologen) Promotors in einer Zelle, insbesondere einer prokaryontischen Zelle exprimiert und anschließend aus der Zelle oder dem Kulturmedium gewonnen wird. Diese heterologe Expression von Proteinen ist ein dem Fachmann auf dem Gebiet der Molekularbiologie bekanntes Standardverfahren, auf das hier nicht näher eingegangen werden muß. In diesem Zusammenhang wird z.B. auf die Darstellung der Expression klonierter Gene bei Sambrook et al., (Molecular Cloning. A Laboratory Manual, Cold Spring Harbor Laboratory Press (1989), Kapitel 16 und 17) verwiesen.

Ein weiteres bevorzugtes Anwendungsgebiet des erfindungsgemäßen Verfahrens ist die Stabilisierung von nativen Proteinen in gelöstem Zustand. So kann beispielsweise durch Zusatz von "Hsp90" Proteinen eine Konstanthaltung der Empfindlichkeit bei optischen Tests in proteinhaltigen Lösungen erreicht werden, bei denen Störungen durch eine geringe Stabilität von in der Testlösung vorhandenen Proteinkomponenten auftreten können. Ein derartiger optischer Test beinhaltet vorzugsweise eine enzymatische Reaktion, besonders bevorzugt eine enzymatische Reaktion, bei der ATP als Cosubstrat vorhanden ist.

Ein "optischer Test" im Sinne der vorliegenden Erfindung ist ein Bestimmungsverfahren, bei dem eine optische Größe oder die Änderung einer optischen Größe, z.B. Absorption, Transmission, Streulicht etc. gemessen wird. Vorzugsweise wird die vorliegende Erfindung bei Verfahren angewendet, in denen das Streulicht bestimmt wird, z.B. bei turbidometrischen, nephelometrischen oder fluorimetrischen Verfahren.

Weiterhin können auch proteinhaltige Lösungen stabilisiert werden, die Antikörper oder von Antikörpern abgeleitete Derivate enthalten.

Ein weiterer Gegenstand der vorliegenden Erfindung ist auch ein Reagenz zur Stabilisierung von Proteinen in wäßriger Lösung, das fest oder flüssig sein kann und ein oder meherere "Hsp90" Proteine, insbesondere das "Hsp90" Protein aus Rinderpankreas enthält. Ein erfindungsgemäßes Reagenz kann beispielsweise das "Hsp90" Protein in gelöster Form oder/und als Lyophilisat enthalten.

Die Erfindung soll weiterhin durch die folgenden Beispiele in Verbindung mit der Zeichnung erläutert werden. Es zeigt:
- Abb. 1: den Aggregationsverlauf bei einer Renaturierung von Citratsynthase in Abhängigkeit von der "Hsp90" Konzentration,
- Abb. 2: die Reaktivierungskinetik denaturierter Citratsynthase bei 20°C in Gegenwart bzw. Abwesenheit von aktivem "Hsp90", und
- Abb. 3: die Reaktivierungskinetik denaturierter Citratsynthase bei 37°C in Gegenwart bzw. Abwesenheit von aktivem "Hsp90", und
- Abb. 4: die Reaktivierungskinetik denaturierter Fab-Fragmente (MAK33 aus Maus) in Gegenwart bzw. Abwesenheit von aktivem "Hsp90".

### Beispiele

### Materialien: Citratsynthase aus Schweineherz (Boehringer Mannheim GmbH, Best.-Nr. 103 373)

Die Reinigung von "Hsp90" aus Rinderpankreas erfolgte nach Welch und Feramisco (1982), J.Biol.Chem. 257, 14949-14959.

220 g Rinderpankreas wurden mechanisch zerkleinert und homogenisiert. Nach Aufschluß der Zellen wurde das Homogenat bei 140 000xg im Sucrosegradienten differentiell zentrifugiert und der cytosolische Überstand auf einer DE52-Anionenaustauschchromatographiesäule aufgetrennt. Das Hsp90-Eluat (200-240 mmol/l NaCl) wurde dann ein weiteres Mal auf der DE52-Säule chromatographiert. Die Elution von Hsp90 erfolgte zwischen 175 mmol/l und 250 mmol/l NaCl. Im Anschluß daran wurde eine Anionenaustauschchromatographie mit Hydroxylapatit und eine Gelfiltration mit Superose 6 durchgeführt.

Das Fab-Fragment bzw. der gesamte Antikörper MAK33 der Klasse IgG1 aus Maus sind in folgenden Publikationen beschrieben: 1) Buckel, P., Hübner-Parajsz, C., Mattes, R., Lenz, H., Haug, H. & Beaucamp, K., (1987), Gene **51**: 13-19 2) EP-A 0 364 926.

### Beispiel 1

### Einfluß von "Hsp90" auf die Aggregation renaturierender Citratsynthase

Es wurde eine Denaturierung von Citratsynthase durch Inkubation von 15 µmol/l Citratsynthase in 6,0 mol/l Guanidinium·HCl (Gdn·HCl), 20 mmol/l DTE, 50 mmol/l Tris/HCl, 2 mmol/l EDTA, pH 8 für 2 Stunden bei 20°C durchgeführt.

Anschließend erfolgte eine Renaturierung der Citratsynthase in Abwesenheit bzw. in Gegenwart von "Hsp90". Dazu wurde denaturierte Citratsynthase (s.o.) unter Rühren in einem Verhältnis von 1 : 100 in Lo-Puffer (40 mmol/l 4-(2-Hydroxyethyl)-1-piperazinethansulfonsäure (HEPES), 20 mmol/l KOH, 20 mmol/l KCl, 10 mmol/l (NH₄)₂SO₄, 2 mmol/l Kaliumacetat, 0,5 mmol/l EDTA, 1 mmol/l Dithiothreitol (DTT), pH 7,0) mit unterschiedlichen "Hsp90" Konzentrationen verdünnt. Die Aggregation von Citratsynthase (Endkonzentration 0,15 µmol/l) wurde durch Messung der Lichtstreuung bei einer Emissions- und Excitationswellenlänge von 500 nm über die ersten 5 Minuten erfaßt. Die Totzeit beträgt etwa 5 Sekunden. Die Aggregation ist in willkürlichen Einheiten angegeben. Das Gesamtvolumen des Reaktionsansatzes betrug 1500 µl.

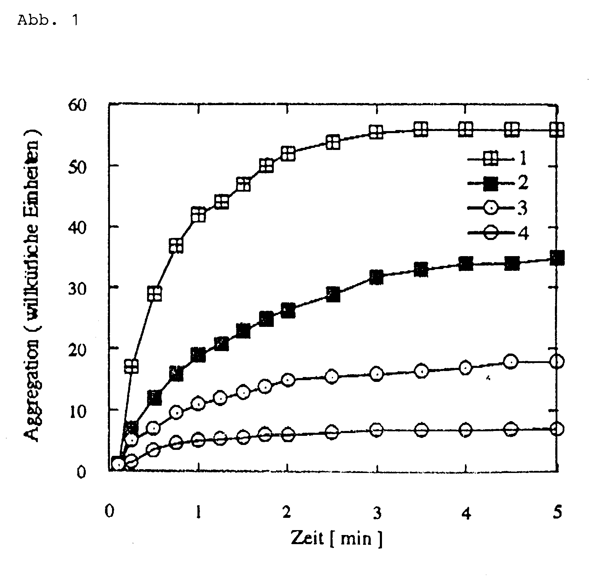

Es wurden insgesamt 4 Reaktionsansätze durchgeführt:
1. Lo-Puffer
2. Lo-Puffer, 0,16 µmol/l Hsp90
3. Lo-Puffer, 0,27 µmol/l Hsp90
4. Lo-Puffer, 0,54 µmol/l Hsp90
Aus Abb. 1 ist ersichtlich, daß mit steigender "Hsp90" Konzentration eine starke Verringerung der Aggregation beobachtet wurde.

### Beispiel 2

### Einfluß von "Hsp90" auf die Reaktivierung von Citratsynthase

Die Denaturierung von Citratsynthase erfolgte wie in Beispiel 1 beschrieben.

Die Reaktivierungskinetik denaturierter Citratsynthase wurde wie folgt bestimmt:
Denaturierte Citratsynthase wurde unter Rühren 1 : 100 in den Reaktivierungsansatz verdünnt (Endkonzentration 0,15 µmol/l) und bei 20°C inkubiert. Nach definierten Zeitpunkten werden dem Reaktionsansatz Aliquots entnommen und zur Bestimmung der Aktivität in den Citratsynthase-Aktivitätstest eingesetzt. Die Aktivität nativer Citratsynthase nach zweistündiger Inkubation bei 20°C wird als 100 % gesetzt. Das Gesamtvolumen der Reaktivierungsansätze betrug 300 µl.

Der Citratsynthase-Aktivitätstest wurde wie folgt durchgeführt:
Citratsynthase katalysiert die Synthese von Citrat aus Oxalacetat und Acetyl-CoA. Dabei wird SH-CoA freigesetzt und die entstehende SH-Gruppe mit Hilfe von Ellman Reagens DTNB (5,5'-Dithio-bis-2-nitrobenzoesäure) durch Freisetzung von gelber 2-Mercapto-5-nitrobenzoesäure nachgewiesen.

| Im Testansatz enthalten: | Konz. der Stammlösung | Konz. im Test |
|---|---|---|
| 10 µl | Oxalacetat (1,37 mg/ml) | 0,10 mmol/l |
| 30 µl | Acetyl-CoA (4,1 mg/ml) | 0,15 mmol/l |
| 10 µl | DTNB (3,96 mg/ml) | 0,10 mmol/l |
| 20 µl | Citratsynthase | |
| 930 µl | 50 mmol/l Tris/HCl, 2 mmol/l EDTA, pH 8,0 | |

Aus der Zunahme der Absorption bei 412 nm über die Zeit läßt sich die spezifische Aktivität der Citratsynthase errechnen.

Es wurden folgende Ansätze durchgeführt:
1. Lo-Puffer, 1 mg/ml Rinderserumalbumin (BSA)
2. Lo-Puffer, 1 mg/ml BSA, 0,15 µmol/l Hsp90
3. Lo-Puffer, 1 mg/ml BSA, 0,15 µmol/l Hsp90 hitzedenaturiert (Inkubation 15 Minuten, 95°C).

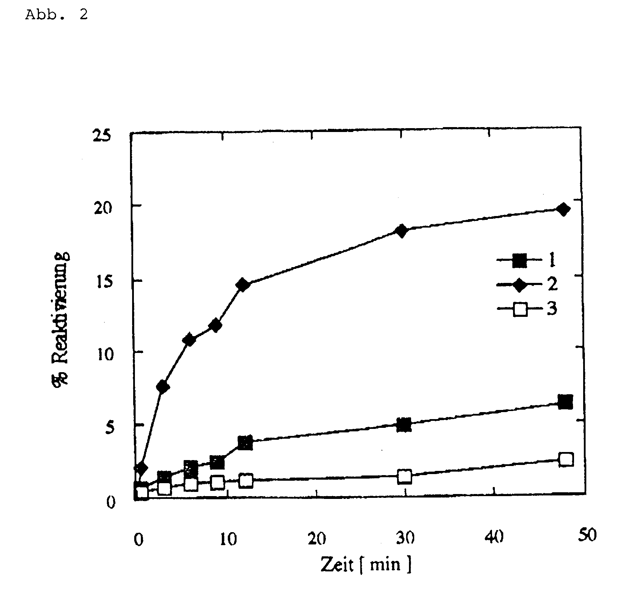

Abb. 2 zeigt, daß nur in Gegenwart von aktivem Hsp90 eine signifikante Aktivitätszunahme der Citratsynthase zu beobachten war.

### Beispiel 3

### Einfluß von Hsp90 auf die Reaktivierung und Stabilisierung von Citratsynthase bei 37°C

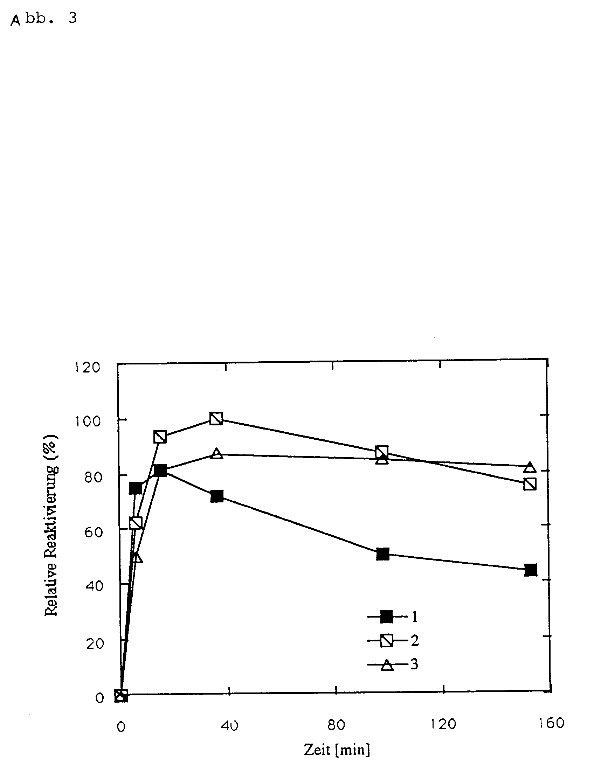

Die Bestimmung der Reaktivierungskinetik erfolgte wie in Beispiel 2 beschrieben, außer daß die denaturierte Citratsynthase bei 37°C in den Reaktivierungsansatz gegeben wurde
Es wurden folgende Ansätze durchgeführt:
1. Lo-Puffer
2. Lo-Puffer, 0,075 µmol/l Hsp90
3. Lo-Puffer, 0,15 µmol/l Hsp90
Abb. 3 zeigt, daß die Citratsynthase ein thermisch instabiles Protein ist. Bereits bei 37°C tendiert das Enzym unter den gewählten Pufferbedingungen zur Aggregation. Die Stabilisierung durch aktives Hsp90 wird in Abb. 3 ersichtlich (vgl. 1 und 3): in Abwesenheit von aktivem Hsp90 nimmt die Reaktivierungsausbeute denaturierter Citratsynthase bereits ca. 20 Minuten nach Beginn der Renaturierung aufgrund der thermischen Inaktivierung der rückgefalteten Citratsynthase ab. In Gegenwart von Hsp90 steigt die Reaktivierungsausbeute der Citratsynthase an und die Inaktivierung der renaturierten Citratsynthase wird in Abhängigkeit der Hsp90-Konzentration zunehmend reduziert. Dies beweist eine Stabilisierung der bereits aktiven Citratsynthasemoleküle durch Hsp90, die neben einer Renaturierungsverbesserung gefunden wird.

### Beispiel 4

### Einfluß von "Hsp90" auf die Reaktivierung denaturierter Fab-Fragmente

### Denaturierung der Fab-Fragmente

Es wurde eine Denaturierung von Fab durch Inkubation von 0,15 µmol/l Fab in 6,0 mol/l Gdn^{.}HCl, 50 mmol/l Tris/HCl, 2 mmol/l EDTA, pH 8,0 für 2 Stunden bei 20°C durchgeführt.

Anschließend erfolgte eine Renaturierung der Fab-Fragmente in Abwesenheit bzw. Gegenwart von "Hsp90". Dazu wurde denaturiertes Fab unter Rühren in einem Verhältnis von 1:100 in den Reaktivierungsansatz verdünnt (Endkonzentration 0,15 µmol/l) und bei 20°C inkubiert.
Nach definierten Zeitpunkten wurden dem Reaktionsansatz Aliquots entnommen und die Reaktion durch Verdünnung des Aliquots in einem Verhältnis von 1:50 in Puffer E (50 mmol/l Tris/HCl, 50 mmol/l NaCl, TX-100, pH 8,0) + Trypsin (30 µg/ml) und Aufbewahrung auf Eis nachweislich gestoppt.

Zur Bestimmung der Reaktivierungsausbeute von Fab wurde ein ELISA (enzyme linked immunosorbent assay) durchgeführt (Buchner, J. & Rudolph, R. (1991), BioTechnology 9: 157-162).

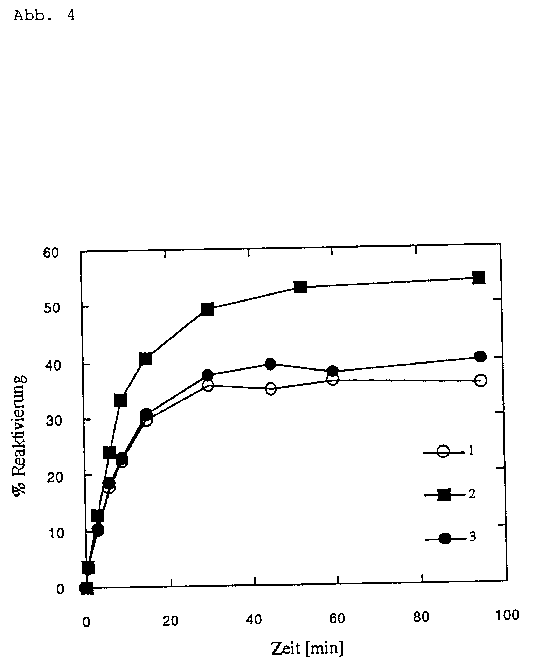

Reaktionsansätze:
1. 50 mmol/l Tris/HCl, 2 mmol/l EDTA, pH 8,0
2. 50 mmol/l Tris/HCl, 2 mmol/l EDTA, pH 8,0, 0,075 µmol/l Hsp90
3. 50 mmol/l Tris/HCl, 2 mmol/l EDTA, pH 8,0, 0,075 µmol/l Hsp90 hitzedenaturiert
   (Inkubation 30 Minuten, 95°C)
Aus Abb. 4 ist ersichtlich, daß die Reaktivierungsausbeute in Gegenwart von aktivem "Hsp90" deutlich zunimmt.

## Patentansprüche

1. Verfahren zur Stabilisierung von Proteinen in einer wäßrigen Lösung,
**dadurch gekennzeichnet,** daß man der wäßrigen proteinhaltigen Lösung ein oder mehrere Vertreter aus der "Hsp90" Proteinfamilie zusetzt.

2. Verfahren nach Anspruch 1,
**dadurch gekennzeichnet,** daß man das "Hsp90" Protein in einem molaren Verhältnis von 0,0001 : 1 bis 10 : 1 bezüglich der zu stabilisierenden Proteine zusetzt.

3. Verfahren nach Anspruch 2,
**dadurch gekennzeichnet,** daß man das "Hsp90" Protein in einem molaren Verhältnis von 0,001 : 1 bis 5 : 1 bezüglich der zu stabilisierenden Proteine zusetzt.

4. Verfahren nach einem der Ansprüche 1 bis 3,
**dadurch gekennzeichnet,** daß das zu stabilisierende Protein ein enzymatisch aktives Protein ist.

5. Verfahren nach einem der vorhergehenden Ansprüche,
**dadurch gekennzeichnet,** daß das zu stabilisierende Protein ein Antikörper oder ein von Antikörpern abgeleitetes Derivat ist.

6. Verfahren nach einem der vorhergehenden Ansprüche,
**dadurch gekennzeichnet,** daß man "Hsp90" aus Rinderpankreas verwendet.

7. Verwendung von "Hsp90" Proteinen zur Unterstützung bei der in vitro-Renaturierung von denaturierten Proteinen.

8. Verwendung von "Hsp90" Proteinen zur Stabilisierung von nativen Proteinen in gelöstem Zustand.

9. Verwendung nach Anspruch 8 zur Konstanthaltung der Empfindlichkeit bei optischen Tests in proteinhaltigen Lösungen, bei denen Störungen durch eine geringe Stabilität von in der Testlösung vorhandenen Proteinkomponenten auftreten können, wobei der optische Test insbesondere eine enzymatische Reaktion beinhaltet.

10. Verwendung nach Anspruch 9,
**dadurch gekennzeichnet,** daß ATP bei der enzymatischen Reaktion als Cosubstrat vorhanden ist.

11. Reagenz zur Stabilisierung von Proteinen in wäßriger Lösung,
**dadurch gekennzeichnet,** daß es ein oder mehrere molekulare "Hsp90" Proteine enthält.

12. Reagenz nach Anspruch 11,
**dadurch gekennzeichnet,** daß das "Hsp90" Protein aus Rinderpankreas stammt.
